(19)

Europäisches Patentamt

European Patent Office

Office européen des brevets

(11) **EP 1 096 411 A2**

## (12) EUROPEAN PATENT APPLICATION

(43) Date of publication:
**02.05.2001 Bulletin 2001/18**

(51) Int. Cl.⁷: **G06F 19/00**

(21) Application number: **00203764.6**

(22) Date of filing: **27.10.2000**

(84) Designated Contracting States:
**AT BE CH CY DE DK ES FI FR GB GR IE IT LI LU MC NL PT SE**
Designated Extension States:
**AL LT LV MK RO SI**

(30) Priority: **29.10.1999 US 162456 P**

(71) Applicants:
- **SMITHKLINE BEECHAM CORPORATION**
  **Philadelphia Pennsylvania 19103 (US)**
- **SMITHKLINE BEECHAM PLC**
  **Brentford, Middlesex TW8 9EP (GB)**

(72) Inventors:
- **Hannenhalli, Sridhar**
  **Rockville, MD 20850 (US)**
- **Russell, Robert B.,**
  **Smithkline Beecham Pharm.**
  **Harlow, Essex CM19 5AD (GB)**

(74) Representative:
**Blakey, Alison Jane et al**
**Smithkline Beecham,**
**Corporate Intellectual Property,**
**Two New Horizons Court**
**Brentford, Middlesex, TW8 9EP (GB)**

(54) **Analysis and prediction of protein functional sub-types from protein sequence alignments**

(57)   Here we present a method for analysis and prediction of functional sub-types from multiple protein sequence alignments. Given an alignment and set of proteins grouped into sub-types according to some definition of function, such as enzymatic specificity, the method identifies positions that are indicative of functional differences by subtraction of sequence profiles, and analysis of positional entropy in the alignment.

EP 1 096 411 A2

**Description**

**Scope of the Invention**

This invention relates to new methods for defining and predicting details regarding function in large and diverse proteins sequence families. This invention presents a method for analyzing and predicting functional sub-types from multiple protein sequence alignments.

**Background of the Invention**

Multiple sequence alignments are central to protein classification and analysis. When protein sequences are aligned, it becomes possible to see sequence conservation patterns that are indicative of, for example, enzyme active sites and secondary structure types (e.g. Zvelebil et al, 1987). With such patterns, it is possible to derive motifs that encapsulate the features defining the protein family. Moreover, the aligned sequences can be used to construct sensitive profiles (e.g. Gribskov et al. 1987), or hidden Markov models (HMMs; e.g. Eddy, 1998, Krogh et al, 1994.) that can be used to detect further, more remote members of a protein family. These techniques and others have aided greatly the construction of protein alignment databases, such as SMART (Schulz *et al*, 1997) and PFAM (Bateman *et al,* 1998), which are of growing importance in the analysis of data from large-scale genome sequence projects.

However, the detection and alignment of diverse protein families creates new problems. Among these is the fact that homologous proteins frequently evolve different functions, which are hereafter refer to as a *sub-type*. It is common for proteins to evolve slightly different functions, such as different substrate specificities, or activities. In extreme cases, both enzymes and effector molecules (i.e. non enzymes) can reside in the same homologous superfamily (Murzin, 1993), and ultimately proteins with similar folds can perform completely different functions (e.g. Russell et al, 1998). If a protein is of unknown function, but is found to belong to a diverse protein superfamily or fold with multiple functions then determining sub-type becomes of great importance.

Often a perfect division of a protein family into sub-types can be accomplished by a simple phylogenetic analysis. In other words: sub-type correlates exactly, and clearly, with the branches of a phylogenetic tree, thus making the prediction of sub-type simply a matter of deciding into which branch a protein belongs. Not surprisingly, most previous attempts to classify proteins have been very reliant on phylogenetic trees.

However, the division of proteins into sub-types can not always be accomplished by phylogeny. If much time has passed since the evolution of different sub-types, then sequences may have diverged beyond the point where phylogeny gives a clear sub-type division. In addition, proteins usually have multiple features that co-evolve, such as differing affinities for more than one substrate, variations in sub-cellular location (e.g. membrane attached verses cytosolic) or the interaction with other proteins that differ across paralogues, even if other details such as catalytic mechanism remain unchanged. Lastly, there remains the possibility that particular details of molecular function may evolve convergently (e.g. Makarova & Grishin, 1999). This is particularly likely in instances where specificity is conferred by only a handful of residues, or even a single position.

Various methods have been developed previously that attempt to address the problem of the analysis and prediction of protein sub-types from protein sequence alignments. Livingstone & Barton (1993) developed a method to annotate protein sequence alignments with the aim of highlighting positions of residue conservation. They made use of the amino acid properties of Taylor (1986) and "sensible groups" provided from sequence similarity, functional or evolutionary criteria to highlight positions in the alignment conferring the unique features of a sub-group. The method was demonstrated graphically by analysis of SH2 and annexin domains it has not been applied to the problem of predicting sub-types.

Casari et al (1995) used a principle component analysis of a vector representation of sequences in space to develop an elegant method for identifying functional residues on proteins based on a multiple sequence alignment. Analysis of various dimensions in the vector sequence space gave both positions that are conserved across the whole protein family, in addition to residues specific to sub-types, either specified in advance, or determined from analysis of the sequence space itself. The method was successful at identifying positions determining specificity in the Ras-Rab-Rho superfamily, SH2 domains and cyclins. Subsequent studies have applied this method to alcohol dehydrogenases (Atrian et al, 1998), the ran-RCC1 interaction (Azuma et al, 1999), effector recognition by GTP-binding proteins (Bauer et al, 1999), and other families.

Lichtarge et al. (1996) developed the "Evolutionary Trace" method, to determine important positions on protein sequences and structures that were of functional importance. Their method combined knowledge of protein structures with an evolutionary history derived from a phylogenetic tree to extract functionally important residues to identifying functional interfaces on protein surfaces. They made the important distinction between positions *conserved* across all sequences and those that vary only between subgroups (*class-specific*). In this way they were able to identify positions on protein structures that were important, both for features of the family as a whole, as well as for particular sub-types. The method has been applied to several protein families, including SH2, SH3, nuclear hormone receptors (Lichtarge *et al*, 1996a), G-proteins/G-protein coupled receptors (Lichtarge *et al*, 1996b) and zinc binding domains (Lichtarge *et al*, 1997).

This invention presents a new approach for analysing and predicting sub-types from protein sequence alignments. Given a multiple sequence alignment and a classification of different sub-types (e.g. differences in enzyme specificity), the *profile difference* method exploits the differences between hidden Markov model profiles to highlight positions on the sequences that are most discerning of each sub-type. The method is insensitive to conservative substitutions, and tolerates missing data by combining alignments with amino acid exchange matrices via the construction of an HMM (Eddy, 1998). For new sequences known to be homologous to an existing family, but of unknown sub-type, the method can exploit the known sub-type classifications and associated profiles to predict sub-type.

## Summary of the Invention

In its primary aspect this invention relates a process for locating positions in a protein alignment that discriminate between different sub-types, which process comprises, in a computational device, in a given alignment $A$ of sequences in family $F$, and having sub-types $S_1$, $S_2$, • • •, $S_k$ of the sequences, extracting the sub-alignment $A^j$ from $A$, corresponding to the sequences of sub-type $S_j$.

## Description of the Figures

Fig. 1. An alscript figure showing an alignment of representatives of nucleotidyl cyclases with positions predicted to confer specificity to adenylate or guanylate highlighted by the method.

Fig. 2. A rasmol figure showing positions found to confer specificy for adenylate or guanlyate in the structure of adenylate cyclase by the instant method.

Fig. 3. An alscript figure showing an alignment of representative protein kinases.

Fig. 4. A rasmol figure showing the structure of cAMP-dependent serine threonine kinase with positions conferring specificity for serine/threonine or tyrosine.

Fig. 5. An alscript figure shows an alignment of representatives of lactate and malate dehydrogenases with positions predicted to confer specificity lactate or malate.

Fig. 6. An alscript figure shows an alignment of representatives of tyrpsin-like serine-proteases with positions predicted to confer specificites known for trypsins, chymotrypsins or elastases.

## Specific Embodiments

The increasing size and diversity of protein sequence families requires new methods to define and predict details regarding function. This invention presents a method for analyzing and predicting functional sub-types from multiple protein sequence alignments. Given an alignment and set of proteins grouped into sub-types according to some definition of function, such as enzymatic specificity, the method identifies positions that are indicative of functional differences by subtraction of sequence profiles, and analysis of positional entropy in the alignment. Alignment positions with significantly low positional entropy are shown to correlate with those known from experiment to be involved in defining sub-types for nucleotidyl cyclases, protein kinases, lactate/malate dehydrogenases and trypsin-like serine proteases. The method is also able to predict sub-type for unclassified sequences. For a threshold of 30% identity, the sub-type method disclosed herein gave an accuracy of 96% compared to 80% obtained by a simple sequence similarity based method over the four families combined together. When applied to a dataset of 42 sub-type groupings derived from an automated parsing of the PFAM and SWISSPROT databases, the method gives an accuracy of 94% compared to 68%.

In invention presents the sub-profile difference method for assigning and analysing sub-types within protein sequence alignments. The four examples set out below show that the method of this invention is better able to detect positions known to confer specificity in close agreement with experiment. Both in these four examples, and the 42 groupings derived from PFAM/SWISSPROT, the method of this invention is shown to predict protein sub-types with remarkable success even in the absence of closely related sequences of the same sub-type. And predictions are much better than those made by a simple sequence comparison.

The method of this invention has many potential applications. For studies on individual proteins from a large sequence family, some prior knowledge of the functions of the sub-types can be used to extract regions on the protein sequence that are the best candidates for laboratory experiments to elucidate function. If an uncharacterized protein shares only a weak degree of sequence similarity with a large protein family then the method can identify the correct sub-type. This is likely to be of greatest use when there are multiple "orphan" members of a protein family and where some priority or rank order of experiments (e.g. ligand binding assays, etc.) must be assigned to keep laboratory experimental effort to a minimum.

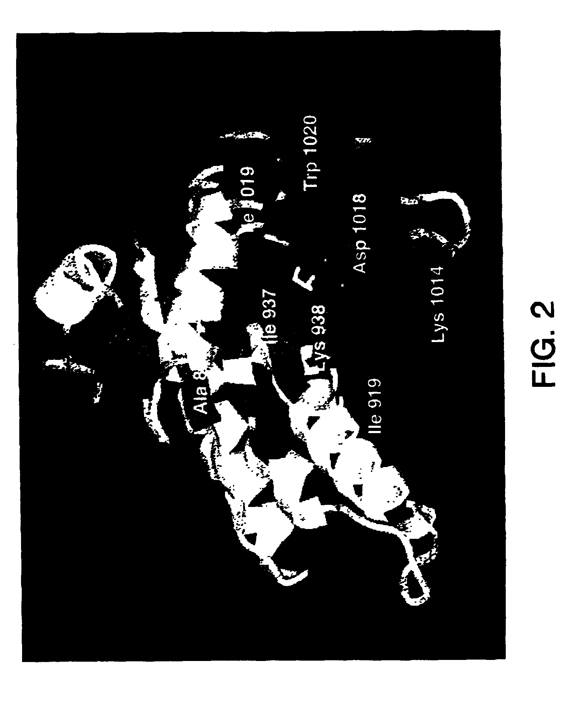

The method may be applied to genome annotation. Newly sequenced genes that are only weak matches to large protein families with differing or varied functions can be tested and highlighted if they contain amino acids that determine a particular sub-type. In this way, it might be possible to avoid ambiguities that arise when a weak sequence similarity score cannot distinguish between two or more different sub-types (e.g. amino acid permeases; see Figure 2a of Brenner, 1999).

Another application is in the prediction of spatial proximity of residues within proteins of unknown 3D structure. There have been several studies attempting to correlate intra-protein distances with correlated mutations (or compensating changes, or cooperative substitutions, or correlated changes; or complementary changes; Gobel et al, 1994; Taylor & Hatrick, 1994; Neher, 1994 Olmea & Valencia, 1994; Russell & Barton, 1994). Although techniques vary, the common theme is to identify positions within a protein sequence that are co-varying during evolution. Residues involved in conferring sub-type are frequently near to each other in space, even when they are far apart on the protein sequence. Methods that identify positions that confer sub-type are thus likely to be of use in predicting inter-and intra-protein distances (e.g. Pazos et al, 1997).

The following abbreviations are used herein:

3D, three-dimensional; GPCR, G-protein coupled receptor; URL, Universal Resource Locator; SCOP, Structural Classification Of Proteins; PFAM, Protein FAMilies; SMART, Simple Modular Architecture Resource Tool; NCBI, National Center for Biotechnology Information; PDB, Protein Data Bank; HMM, Hidden Markov Model. The standard one- and three-letter abbreviations for the amino acids are also used throughout.

## Assessing the discerning value of amino acid positions

The method of this invention, a procedure one might call it, locates positions in a protein alignment that are best able to discriminate different sub-types. Essentially this involves finding positions that are conserved within each sub-type, but that vary between the different sub-types.

Given an alignment $A$ of sequences in family $F$, and the sub-types $S_1, S_2, \cdots, S_k$ of the sequences, one extracts the sub-alignment $A^j$ from $A$, corresponding to the sequences of sub-type $S_j$. The *hmmbuild* program of the HMMER 2.1.1 (http://hmmer.wustl.edu) suite was used to build profile $P^j$ of the alignment $A^j$. The profile at position $i$ of the alignment is represented by $P^j_i$ and the profile value for amino acid $x$ at position $i$ of the alignment is represented by $P^j_{i,x}$. The score profile in the *hmmbuild* output is converted into probability profile such that

$$P^j_{i,x} = 1$$
$$\text{\textit{for all x}}$$

for each alignment position $i$. For a sub-type $s$, one can use $\bar{s}$ to denote the union of all the sub-types excluding s. To estimate the role of an alignment position (or, site) $i$ in determining the sub-type $s$, one can compute the *relative entropy* ( Shannon and Weaver 1963, Durbin et al 1998) of the position i for sub-type s with respect to the entropy of that position for the sub-type $\bar{s}$. Let $RE^s_i$ be relative entropy of $P^s_i$ with respect to $P^{\bar{s}}_i$.

$$RE^s_i = \sum_{\text{\textit{for all x}}} P^s_{i,x} \log \frac{P^s_{i,x}}{P^{\bar{s}}_{i,x}}$$

To estimate the role of an alignment position $i$ in determining the sub-types, one can define *cumulative relative entropy* $CRE_i$ as

$$CRE_i = \sum_{\text{for all sub-types } s} RE^s_i.$$

The cumulative relative entropies for all the positions are converted into *z-scores* based on the distribution of entropies for an alignment. Let μ and σ be the mean and the standard deviation of cumulative relative entropies of all positions, then the *z-score* for position $i$ is computed as:

$$Z_i = \frac{CRE_i - \mu}{\sigma}$$

A position with high *z-score* is expected to be important in determining the sub-types. Inspection of alignments together with knowledge of residues determining specificity via experiment suggested that z-scores > 3.0 correlated well with preconceptions of positions known to determine specificity. This value was thus used in the discussion of individual families below.

Once one identifies the important sites, one needs to identify the residues responsible for the low entropies at those sites. This can be done by computing the *profile difference* of the position i for sub-type *s* with respect to the entropy of that position for the sub-type $\bar{s}$. Let $PD^s_{i,x}$ be value of amino acid $x$ in the difference profile of $P^s_i$ with respect to $P^{\bar{s}}_i$.

$$PD^s_{i,x} = \frac{P^s_{i,x}/P^{\bar{s}}_{i,x}}{P^s_{i,y}/P^{\bar{s}}_{i,y}} \quad \text{for all } y$$

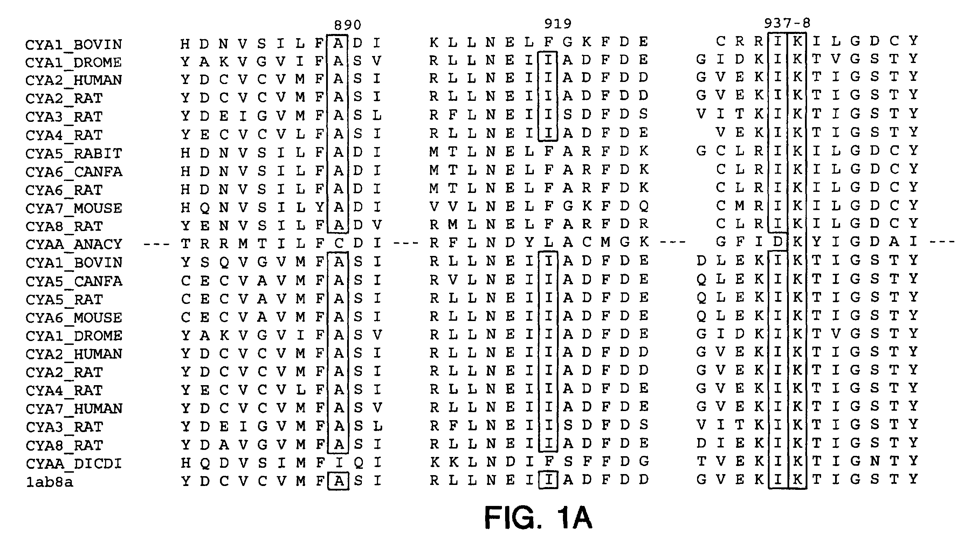

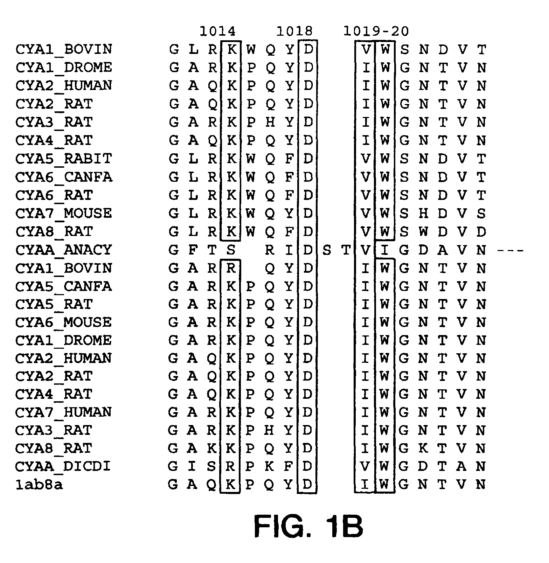

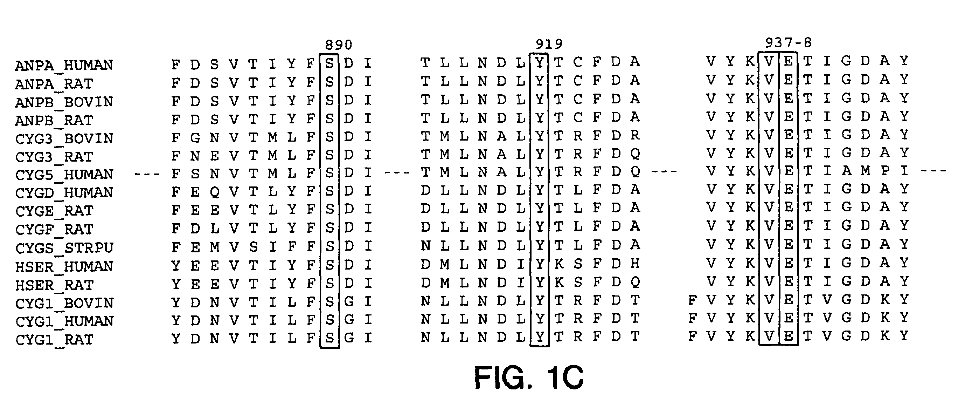

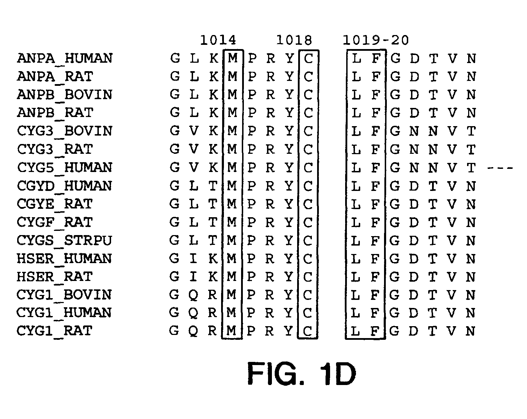

Inspection of results showed that single amino acids (or groups with similar properties) having $PD^s_{i,x} >= 0.5$ agreed with one's prior knowledge of determinants of sub-type specificity. One can thus use this value when highlighting amino acids in Fig. 1, 2, 5 and 6.

**Predicting protein sub-types**

Similarity-Based Method

If a particular sequence $X$ of unknown sub-type has a high degree of sequence similarity to a sequence with known sub-type, and then $X$ can often be assigned (accurately) the same sub-type as the most similar sequence. For comparison to the methods described below, herein is provided a simple *similarity based method* based on this principle. Given a sequence of unknown sub-type, one can assign it the same sub-type as the sequence of known sub-type with the highest percent sequence identity (calculated by ignoring gaps). This method attempts to simulate predictions of sub-type that might be made by a simple sequence database search, or phylogeny.

B. HMM based Method

Since the HMMER method was used to compute the profiles for each sub-type, it was decided to use the alignment program hmmalign to align the sequence of unknown sub-type to all HMMs and assign it to the sub-type yielding the maximum alignment score.

C. Profile (HMM-derived) based Method

Frequently the alignment resulting from *hmmalign* is slightly different from the original alignment. For many protein families, hand-editing is performed to give alignments that are generally better than those generated automatically (e.g. SMART, Schultz et al, 1998). Slight changes to the alignment introduced by an alignment algorithm (i.e. *hmmalign*) might thus affect the prediction accuracy adversely. To avoid this potential problem profile-based method described below was developed.

The assumption is made that a sequence with unknown sub-type is aligned to the other members of the family so that the length of the aligned sequence is the same as the length of the profile. For a sequence $X = x_1 x_2 \cdots x_n$, and profile $P$, the score of $X$ with respect to $P$ is computed as the probability of the sequence given that it is generated by the HMM that the profile corresponds to (or simply, generated by the profile).

$$p(X|P) = \prod_{i=1}^{n} P_{i,xi}$$

To assign a sequence $X = x_1 x_2 \cdots x_n$ with unknown sub-type to one of the sub-types $S_1, S_2, \cdots, S_k$, the probability was computed that the profile generating the sequence corresponded to sub-type $S_i$, given the sequence. In other words, $p(P^i | X)$ for each $i$. was computed from Bayes rule,

$$p(P^i | X) = \frac{p(P^i) \cdot p(X|P^i)}{p(X)}$$

Assuming that each sequence must belong to one of the sub-types, the probabilities were normalized to get the following

$$p(P^i | X) = \frac{p(P^i) \cdot p(X | P^i)}{\underset{\text{for all } j}{p(P^j) \cdot p(X | P^j)}}.$$

Assuming that all sub-types are equally likely, we get the following

$$p(P^i | X) = \frac{p(X | P^i)}{\underset{\text{for all } j}{p(X | P^j)}}$$

The sequence X is assigned to sub-type $S_i$ that maximizes $p(P^i | X)$ (same as maximizing $p(X | P^i)$). The difference between the maximum probability and others can be used as a measure of confidence in the assignment. This will be of importance if a new sequence does not belong to one of the known sub-types. An investigation into such a means to assign confidence to the predictions is in progress.

<u>Sub-Profile based Method</u>

This method is a slight variant of the profile-based method. The difference is that only those positions in the alignment with a positive relative entropy Z-score are used when computing the score of a sequence against a profile. Essentially, this removes contributions of the non-discriminating alignment positions to the score, thus filtering out the noise.

**Evaluation of sub-type prediction accuracy**

It was believe that if a sequence $X$, with unknown sub-type, has a high degree of sequence similarity to a sequence with known sub-type, then $X$ could be assigned the same sub-type. If a very similar sequence of known sub-type were found, then generally the best prediction of sub-type would be a straightforward transfer. The methods disclosed in this invention are aimed at predicting sub-types in the absence of very similar sequences of known sub-type. Thus when evaluating the methods, one first eliminates all the sequences highly similar to $X$. Then one defines sequence similarity as percentage sequence identity (ignoring gaps) and varies the threshold for sequences to ignore when making a prediction.

For the *similarity-based method,* one assigns sequence X to the sub-type of the most similar sequence in the reduced set (after removing the close homologues). For the *HMM based method,* one constructs HMMs using *hmmbuild* for the reduced set of each sub-type, align the sequence to each of the HMMs using *hmmalign* and assign the sequence to the sub-type yielding the maximum score. The *profile based method* and the *sub-profile based method* are similar to the above, but instead of aligning the sequence against the HMMs using *hmmalign,* one simply scores the sequence against the profiles derived from the HMMs, as described before, leaving the original alignment of the sequence un-perturbed.

Aligned sequence data and sub-types of well understood protein families

To test the method, four examples of large enzyme families with clear sub-types were chosen: nucleotidyl cyclases, eukaryotic protein kinases, lactate/malate dehydrogenases and trypsin-like serine proteases. For all of these families laboratory experiments (e.g. site-directed mutagenesis or crystallography) or manual analysis of the alignments have been used previously to determine details regarding specificity. Other examples were sought where phylogeny or simple sequence comparison would not always lead to a correct prediction of catalytic sub-type. For all four of these protein families, a simple phylogeny (performed using bootstrapped trees within the Clustalw package) failed to separate the sub-types into distinct clades.

The aim for these four protein families was to see if previously identified positions were found by the method of this invention. To that end, it was required that all of the examples contained at least one protein of known 3D structure to allow inspection of spatial proximity of amino acids thought to be important. Unless otherwise stated alignments were taken from PFAM (Bateman et al, 1998) and groupings from inspection of SWISSPROT annotations or prior biochemical knowledge.

For a rigorous assessment ideally one would require a carefully curated set of sub-groupings for a large set of alignments (e.g. from PFAM or SMART). It is problematic to construct a large set of test examples automatically since details regarding molecular function of a particular protein are not easily extracted from any database currently available. However certain resources do provide some capacity to derive such a large set of alignments and sub-type groupings. Herein proteins were divided within the PFAM database (version 4.0, 1465 alignments) by considering functional details described in SWISSPROT. The experiment sought to use all keyword data (KW). However these produced ambiguities that lead to a vast number of meaningless groups. The focus was then shifted to details of enzymatic activity. Activities were extracted by searching for the string "CC -!- CATALYTIC ACTIVITY" for each SWISSPROT entry in PFAM alignments, and then grouping sequences in alignments accordingly. After ignoring groups with fewer than ten sequences, all possible group combinations were constructed, ignoring those where sequences were contained in more than one group.

This procedure initially produced 96 groups from 50 alignments. Inspection ruled out 18 (e.g. similar but not identical activities; knowledge that enzymatic function was not conferred in the domain considered). Another 16 were ignored because the pairwise sequence identities indicated that the divisions based on catalytic activity would be easily discernable by phylogeny. Thus 62 groupings from a total of 42 alignments remained. These are described in Table 1. To avoid biasing due to multiple groups from a single alignment, one grouping was selected from each alignment. This resulted in a set of 42 groupings over 42 alignments.

Table I

Groupings for PFAM alignments extracted from SWISSPROT

| No. PFAM name | Substrates |
|---|---|
| 2-oxoacid_dh | acetyl-coA / succinyl-coA |
| ATP-gua_Ptrans | creatine / L-arginine |
| Aconitase_C | 3-isopropylmalate / maleate / citrate |
| Epimerase | dTDP-glucose / UDP-glucose |
| FGGY | D-xylulose / glycerol |
| GATase | 1-(2-carboxyphenylamino)-1-deoxy-D-ribulouse-5-phosphate /2ATP + glutamine /ATP + xanthosine-5'-phosphate + L-glutamine /chorismate + L-glutamine |
| GATase_2 · | 5-phospho-$\beta$-D-ribosylamine + L-glutamate / ATP + L-asparate + L-glutamine / |

|  | L-glutamine + D-fructose-6-phosphate |
| GHMP_kinases | D-galactose / L-homoserine |
| HMA | ATP / HG+NADP(+)+H(+) |
| OTCace | Asparate / Ornithine |
| Orn_DAP_Arg_deC | L-Arginine /L-ornighine / meso –2,5diaminoheptanedioate |
| PDEase | (Adenosine / Guanosine) -3',5'-cyclicphosphate |
| PGAM | 2-phosphoglycerate + 2,3-diphosphoglycerate / |
|  | ATP + D-fructose-6-phosphate / |
|  | D-fructose-2-6-bisphosphate |
| PGM_PMM | α-D-glucose-1-phosphate / D-mannose-1-phosphate |
| Pribosyltran | IMP / orotidine-5'-phosphate |
| Rhodanese | Protein-Tyr-phosphate / thiosulfate + cyanide |
| Rieske | Plastoquinol-1 + 2 oxydised plastocyanin / |
|  | $QH_2$ + 2 ferricytochrome C |
| SQS_PSY | 2-farnesyldiphosphate / prephytoenediphosphate |
| S_T_dehydratease | L-theronine / O-acetyle-L-serine |
|  | O-phospho-L-homoserine |
| Semialdhyde_dh | L-asparate-semialdehyde / |
|  | N-acetyle-L-glutamte-5-semialdehyde |
| aakinase | L-aspartate / L-glutamate / L-glutamate-5-semialdehyde |
| aconitase | 3-isopropylmalate / citrate |
| adh_zinc | alcohol / cinnamylalcohol |
| aminotran_1 | L-asparate + 2-oxoglutarate / S-adeosylmethionine |
| aminotran_3 | (S)-4-amino-5-oxopentanoate / |
|  | 4-aminobutanoate + 2-oxoglutarate / |
|  | L-ornithine + A 2-oxoacid / |
|  | N-2-acetyl-L-ornithine + 2 oxoglutarate |
| cytochrome_b_N | Plastoquinol-1 + 2 oxydised plastocyanin / |
|  | $QH_2$ + 2 ferricytochrome C |
| guanylate_cyc | ATP / GTP |
| malic | NAD / NADP |
| isodh | 3-carboxy-2-hydroxy-4-methylpentanoate / |
|  | Isocitrate + NAD / |
|  | Isocitrate + NADP |
| ldh | L-lactate / L-malate |

| ligase-CoA | ATP / GTP |
| lyase_1 | L-argininosuccinate / L-malate |
| oxidored_nitro | reduced ferredoxin + 6H+ + N2 +NATP |
| | chlorophyllidea + NADP |
| oxidored_q1 | plastoquinone / ubiquinone |
| oxidored_q1_N | plastoquinone / ubiquinone |
| pyr_redox | dihydrolipoamide |
| | $Hg + NADP + H^+$ / NADPH + glutathione |
| pyridoxal_deC | L-glutamate /L-tryptophan |
| tRNA-synt_1 | L-isoleucine / L-leucine / L-methionine / L-valine |
| tRNA-synt_1b | L-tryptophan / L-tyrosine / L-asparagine |
| tRNA-synt_2 | L-aspartate / L-lysine / L-histidine |
| tRNA-synt_2b | L-histidine / L-proline / L-serine / L-threonine |
| tyrosinase | 2-catechol / L-tyrosine + L-dopa |

Substrates for different sub-types are separated either by a `/' character or a line-break.

A problem with the above procedure is the assignment of catalytic activity to the correct domain. For example, the groupings for SH2 domains were discarded as it was known that the kinase catalytic activity was not localised in this domain. There is no simple way of doing this by parsing SWISSPROT, thus the results of analysing these data must be considered with some caution.

**Results**

Nucleotidyl cyclases

Nucleotidyl cyclases are a family of membrane attached or cytosolic domains that catalyse the reaction that forms a cyclic nucleotide monophosphate from a nucleotide triphosphate. The known cyclases act either on GTP (guanalyate cyclase) or ATP (adenylate cyclase). Mutations of two residue positions from Glu - Lys and Cys - Asp are known to be sufficient to change the specificity of the enzyme from GTP to ATP (Tucker et al, 1998). Mutation of several other residues near to the key Cys-Asp change were shown not to have any significant effect on specificity or enzymatic activity.

Figure 1 shows an alignment of nucleotidyl cyclases highlighting positions that have an entropy Z > 3.0. These positions are shown on the known 3D structure of adenylate cyclase (Zhang et al, 1997: PDB code 1ab8) in Figure 2. The first and third best positions are the Asp-Cys (residue 1018 in 1ab8, Z=6.5) and Lys-Glu (938, Z=4.0) changes identified by Tucker et al (1998) that can be changed to modify cyclase specificity. Positions 1019 (Z=2.6) and 1020 (Z=3.9) were also identified by Tucker et al, but the change from Leu, Phe in Guanylate cyclase to Ile,Trp (as is seen in most adenylate cyclases) in concert with the changes above actually lead to a poorer adenylate cyclase activity. The Trp - Phe (1020) change implies that a larger side chain is needed in the Adenylyl cyclases, and changes Ile - Val (937, Z=3.0) and Val/Ile - Leu (1019) appear to be involved in subtle positioning of the residues the are adjacent on the sequence. Positions 937 and 1019 pack against one another in the known structures, implying a complementary change. The Val/Ile - Leu (1019) change is also interesting in that it suggests that the adenylyl cyclases require a branched $C\beta$ residue (i.e. two non-hydrogen substituents on the beta carbon, as is only seen in valine, isoleucine or threonine) instead of the non-$C\beta$-branched leucine found in the Guanylyl cyclases. Inspection of the structure suggests that this may have to do with adopting a slightly different main-chain conformation: branched $C\beta$ residues are slightly

more restricted in the backbone psi/phi conformations that they can adopt (Swindells et al, 1995). These observations may help to explain why the mutants involving position 1019 in Guanylate cyclase (substituting Leu with Ile; in concert with the Glu - Lys and Cys - Asp changes mentioned above) performed by Tucker et al (1998) lead to poorer adenylate cyclase activities.

The method also identified additional positions. The second best scoring position was the Lys/Arg - Met substitution (1014, Z=4.6), which is also far away from the others in space if one considers a single cyclase subunit. However, inspection of the dimeric structure of adenylate cyclase (PDB code 1azs) shows that the equivalent position from the adjacent subunit is in the same location as the other positions discussed above. Changes Ala-Ser (890, Z=3.1) and Ile-Tyr (919, Z=3.6) are in the same approximate spatial location as the others, but are not in direct contact with any of the positions mentioned above. Inspection of the structure suggests that these changes may be responsible for subtle shifts in secondary structures that may help accommodate different substrates. However, only further experimental studies on the cyclases can reveal the meaning of these changes.

**Protein kinases**

Protein serine, threonine and tyrosine kinases are one of the largest protein families known, estimated to make up between 1-2% of proteins from metazoan genomes (e.g. Chervitz et al, 1998). They function to attach a phosphate group to a hydroxyl moiety on a particular amino acid side chain. A major division is that between serine/threonine and tyrosine kinases. Serine and threonine are quite similar in size and shape, with a hydroxyl group attached to the C$\beta$ carbon; the only difference is a methyl group in threonine in place of a hydrogen in serine also attached to the C$\beta$ carbon. In tyrosine, however, the hydroxyl group is attached to six-membered aromatic ring, making both the chemistry of the reaction and the size of the substrate substantially different. Certain positions are known to confer this specificity. Within kinase sub-domain VI of protein kinases (after Hanks et al, 1988), the consensus sequence RDLKPEN is usually found in serine/threonine kinases, whereas the sequence RDLAARN is typical of tyrosine kinases (Hanks & Hunter, 1996). Analysis of the first kinase crystal structures also identified other regions (Taylor et al, 1995). To test the profile difference method on this family of proteins, this work used the alignment (295 sequences) and divisions from the protein kinase resource (Smith et al, 1998). The three major categories of Ser/Thr kinases were grouped into a single type, and the category "other protein kinases" (OPK, of unknown or ambiguous sub-type) were ignored.

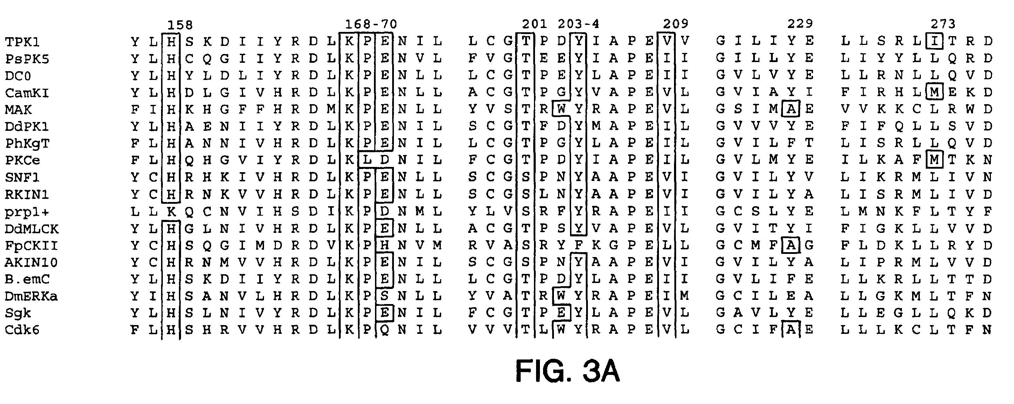

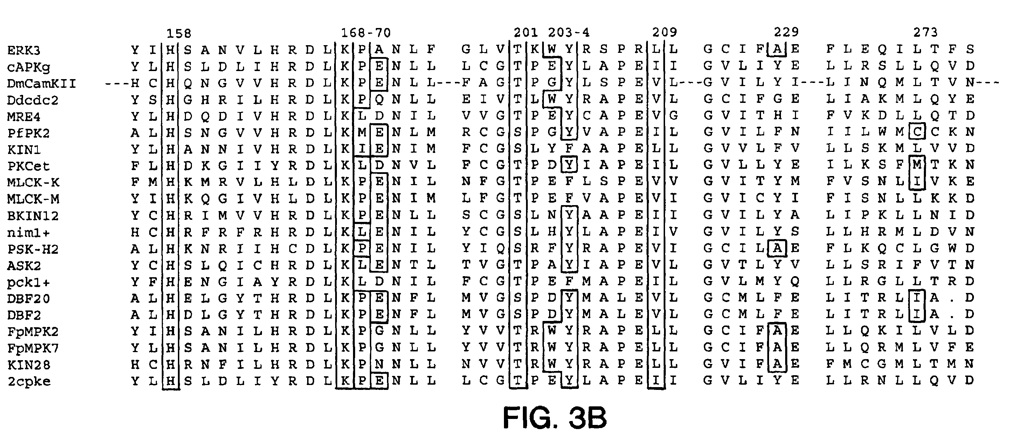

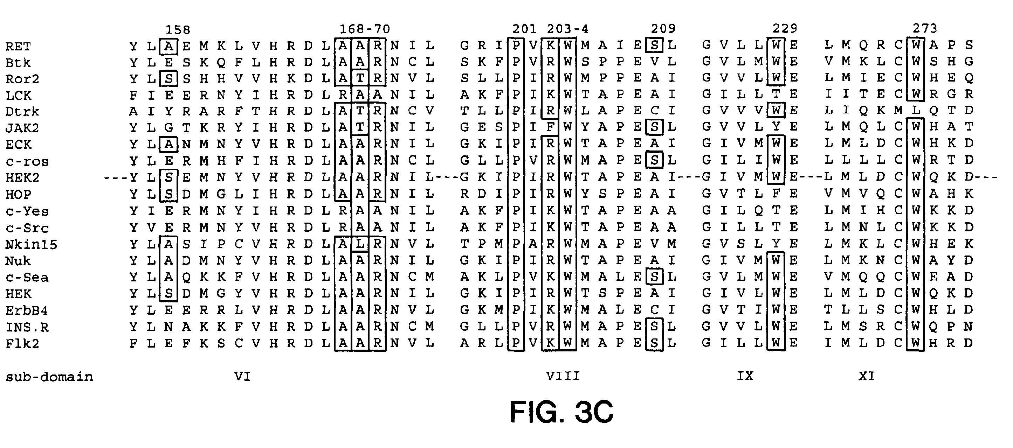

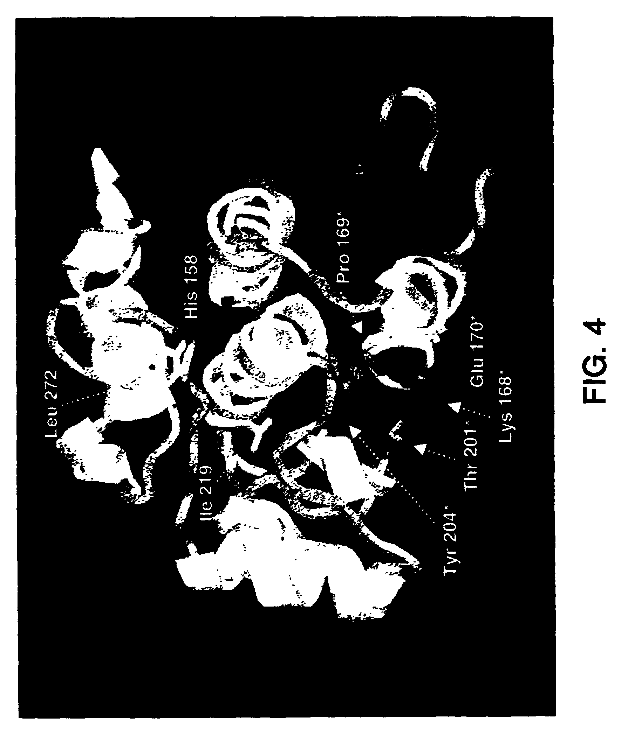

Figures 3 and 4 show an alignment and 3D structure highlighting the ten positions with the highest entropy Z score (Z > 3.04). All of these positions lie in the C-terminal (catalytic domain), and most of the top scoring positions lie in two regions of the sequence and adjacent in space. One of these regions, containing positions 168-170 (in PDB code 2cpk, Z=5.7, 3.4,3.9) are in the Hanks et al sub-domain VI region known to determine kinase specificity (discussed above), in the catalytic loop (Lys,Pro,Glu - Ala,Ala,Arg). The second contains substitutions Thr/Ser - Pro (201, Z=5.5), Trp - Lys/Arg (203, Z=3.2), Tyr - Trp (204, Z=6.5) and Ile/Val/Leu - Ser (209, Z=3.2) from sub-domain VIII, within (or near to) the P+1 loop. Most of the positions within these regions were identified by Taylor et al (1995) as those that are most characteristically distinct for the Ser/Thr and Tyr sub-types.

The other positions shown in Figure 3 are His - Ala/Ser (158, subdomain VI), Ala - - Trp (229, subdomain IX, Z=3.1) and Leu/Met/Cys - Trp (273, subdomain XI, Z=3.3), and numerous other positions with Z>=3.0 are near to these in space (results not shown). None of these positions are close enough to interact directly with those residues discussed above. However, as for the cyclases, inspection suggests that they may be involved in aiding subtle conformational changes of the structure to accommodate differing substrates. Several other differences between Protein kinase A (Ser/Thr) and Insulin receptor tyrosine kinase (IRK) were reported by Taylor et al (1995), though not detected during this study. Inspection of the alignment shows that the positions are either not conserved across the sub-types, or show substantial overlap between the Ser/Thr and Tyr sub-types when one considers all homologous sequences.

Lactate/malate dehydrogenases

Dehydrogenases that act on lactate and malate are part of a larger superfamily of Rossmann fold (nucleotide binding domain) containing enzymes (e.g. Rossmann & Argos, 1976: Russell & Barton, 1992). Lacate and malate dehydrogenases (LDH, MDH) form a large sub-set of this family and share the additional common feature of a similar substrate-binding domain. They are found across all kingdoms of life and are thus highly divergent, meaning that it is difficult to distinguish between lactate and malate sub-types. A key mutation Gln-Arg (position 102 in B. stearothermophilus LDH) is known to switch the specificity from lactate to malate (Wilks *et al*, 1988), and is known to be involved in distinguishing lactate from malate. In addition, all possible variants of positions 101 and 102 have been analysed (Hawrani et al, 1996) and have determined residues conferring substrate specificities for many other substrates known to bind to this large family of enzymes, including phenyllactate, hydroxyisocaproate and 4-phenyl-2-hydroxy-butanoate.

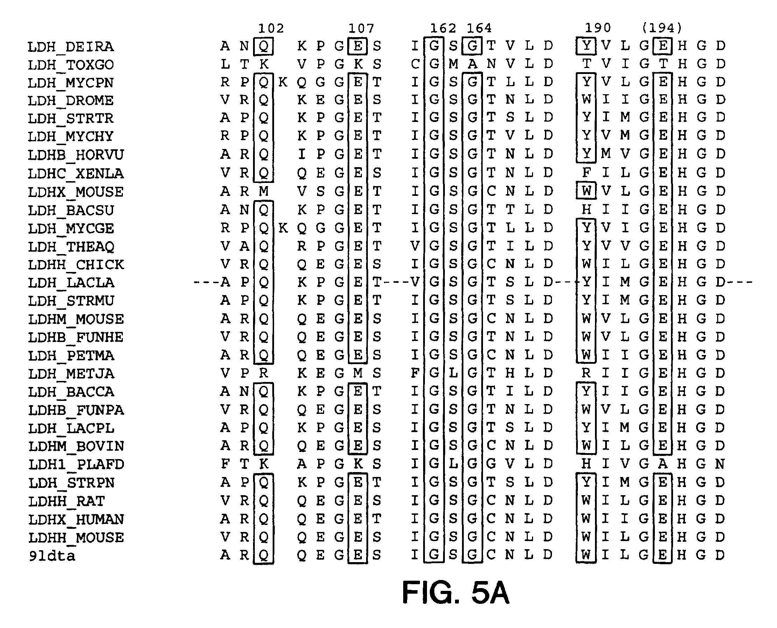

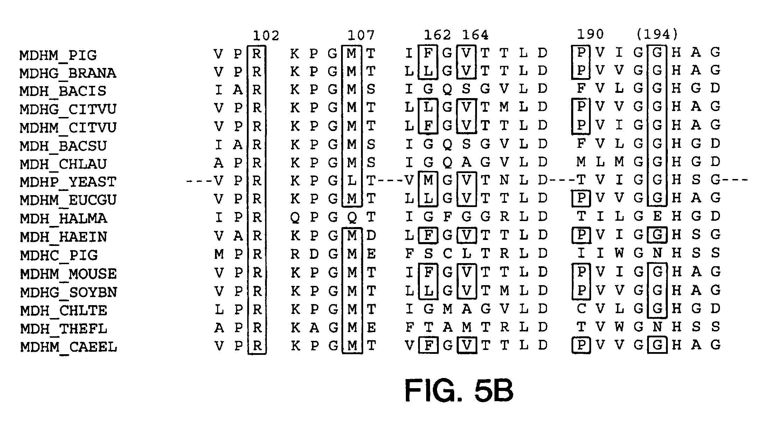

Figure 5 shows an alignment illustrating the 6 positions with the highest entropy Z score. The position with the highest entropy (Z = 4.0) is the Gln-Arg (102) change identified by experiment. With the exception of the Tyr - Pro change (position 190, Z=3.4) all positions are near to the Gln-Arg position and surround the experimentally determined location of NAD. They are thus likely to be involved in the lactate/malate distinction. All have Z>3.0 with the exception of the Glu - Gly change at position 194 (Z=2.9), which is shown as it also appears to have a role in determining substrate specificity.

### Serine proteases

Trypsin-like serine proteases are a large family of enzymes involved in the hydrolysis of peptide bonds. Although they all act via a similar catalytic mechanism, they have different preferences for the amino acids that they prefer to cleave. Trypsin cleaves C-terminal to arginine or lysine, chymotrypsin next to large aromatic residues, and Elastase cleaves next to small uncharged amino acids. It was proposed long ago that the distinction is conferred by key changes in a specificity pocket (e.g. Fersht, 1985). Three positions were proposed originally to define the pocket. An aspartic acid found in trypsin (Asp 189) is usually replaced by a small residue in chymotrypsins (Ser) and elastases (Gly). Two positions adjacent to this in space were originally described as defining substrate differences in these three families (e.g. Fersht, 1985). Positions 216 and 226 (in trypsin) are generally glycines in chymotrypsins and trypsins, but replaced by valine and threonine in elastases.

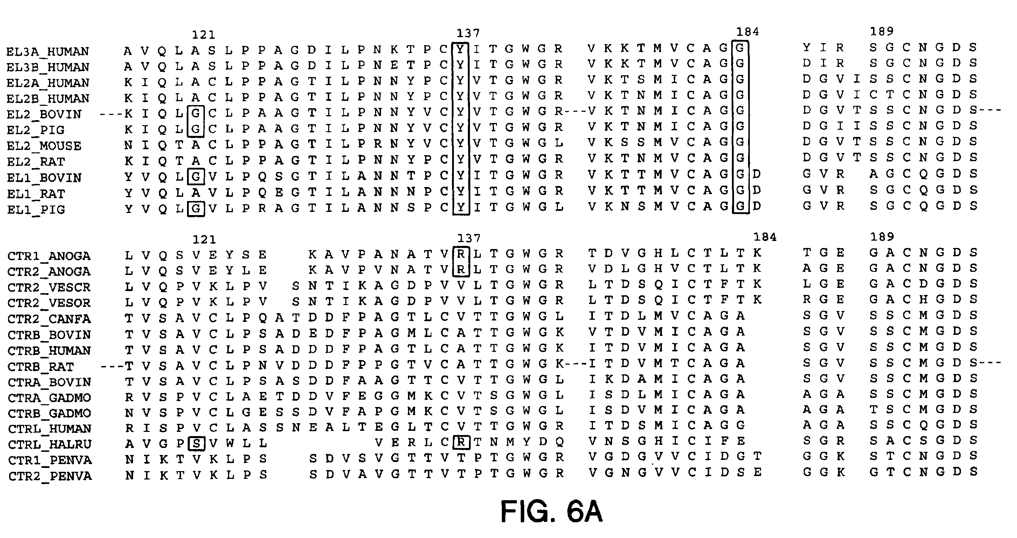

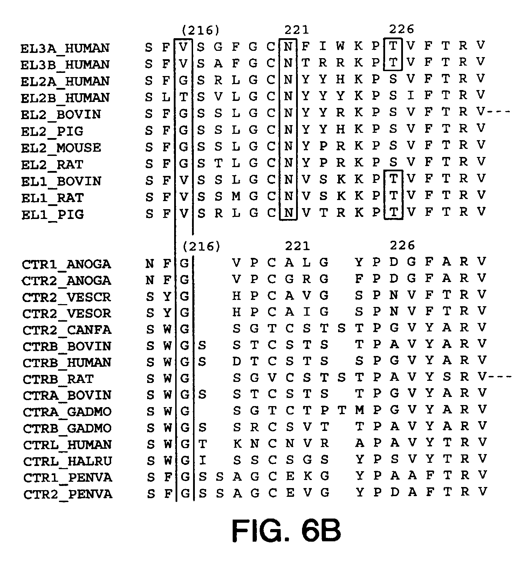

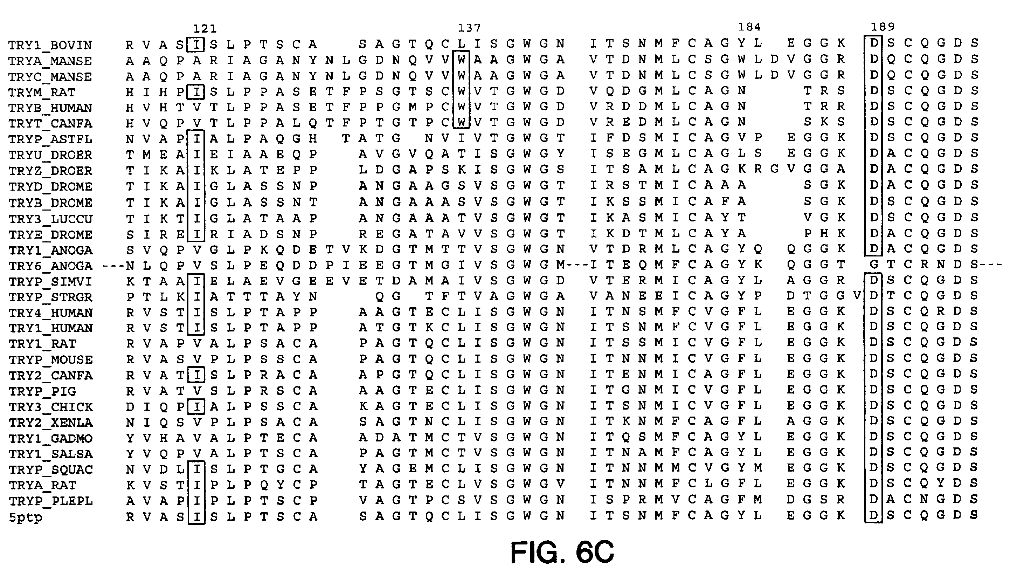

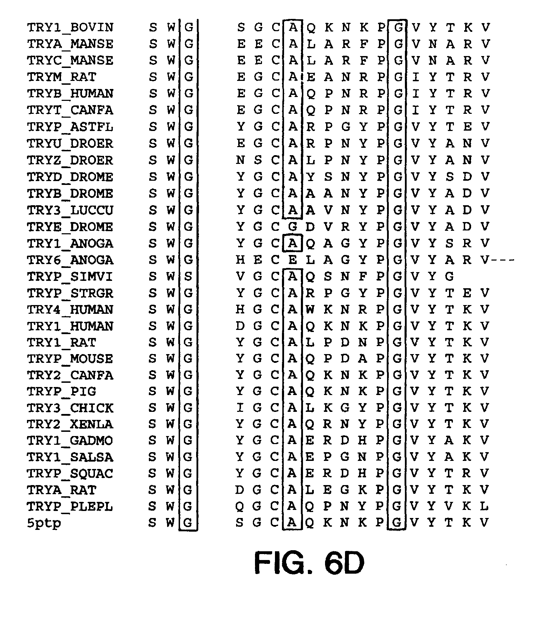

Figure 6 shows the positions with Z>=3.0 identified by the method for the trypsin-like serine proteases when grouped into elastase, chymotrypsin and trypsin sub-types. The top two scoring positions (position 189 in PDB code 5ptp, Z=5.6 and 226, Z=3.9) correspond to two of the pocket positions above. The third pocket position (216) has a low Z score (1.0). Inspection of the alignment (see boxed position in Figure 6) shows that glycine is frequently tolerated in the elastases sub-type, giving a low Z score. The third best scoring position (221, Z=3.6) is an Asn in the elastases, and generally an Ala in trypsins, and is near to the specificity pocket discussed above. Of the other three positions identified only position 184 (Z=3.1) is near to the other pocket forming residues in space. Here glycine, which is preferred in the elastases, may aid the recognition of small side-chains in elastase substrates. The other two positions with Z>3.0 (121, Z=3.5; 137, Z=3.3) are not near to the pocket in space, though like the cyclases and kinases (above), it is possible that they are involved in any subtle conformational adjustments to accommodate differing substrates.

### PFAM alignments as grouped by SWISSPROT

The analysis by PFAM alignments provides a large set of alignments useful for accessing the predictive accuracy of that method (Bateman *et al*, 1998).

### Prediction accuracies

Table 2 gives the prediction accuracies for the 4 families discussed above. The relative accuracies of the *HMM, profile and similarity based methods* vary greatly according to the percentage sequence identity threshold used to include sequences in the alignment. When all but the very distant homologues of the predicted sequence are removed (i.e. 20% threshold) the *HMM* and *profile* methods clearly out-perform the similarity method. The distinction between the methods diminishes as the threshold is raised, and when sequences with identities 50% or greater with the predicted sequence are included in the alignment, the predictive accuracies of the methods are indistinguishable.

Table 2

| Prediction accuracies of all methods for four well characterised protein families | | | | | | | | |
|---|---|---|---|---|---|---|---|---|
| Sequence Identity Threshold (%) | Cyclase (2) 72 | | Kinase (2) 293 | | Dehydrogenase (2) 103 | | Trypsin (3) 101 | |
| | P | A (%) | P | A (%) | P | A (%) | P | A (%) |
| 20 | 6 | SP: 100 HMM: 33 P:0 SS: 0 | 191 | SP: 100 HMM: 94 P:96 SS: 60 | 103 | SP:93 HMM: 78 P:47 SS: 24 | 0 | SP:X HMM: X P:X SS: X |

Table 2 (continued)

| Prediction accuracies of all methods for four well characterised protein families | | | | | | | | |
|---|---|---|---|---|---|---|---|---|
| Sequence Identity Threshold (%) | Cyclase (2) 72 | | Kinase (2) 293 | | Dehydrogenase (2) 103 | | Trypsin (3) 101 | |
| | P | A (%) | P | A (%) | P | A (%) | P | A (%) |
| 30 | 43 | SP: 100 HMM: 91 P: 63 SS: 21 | 293 | SP: 100 HMM: 98 P: 97 SS: 98 | 103 | SP: 93 HMM: 88 P: 88 SS: 63 | 37 | SP: 68 HMM: 65 P: 68 SS: 54 |
| 40 | 63 | SP: 100 HMM: 63 P: 54 SS: 56 | 293 | SP: 100 HMM: 100 P: 100 SS: 100 | 103 | SP: 90 HMM: 92 P: 91 SS: 87 | 89 | SP: 96 HMM: 85 P: 83 SS: 66 |
| 50 | 72 | SP: 100 HMM: 99 P: 99 SS: 97 | 293 | SP: 100 HMM: 100 P: 100 SS: 100 | 103 | SP: 93 HMM: 95 P: 91 SS: 97 | 101 | SP: 97 HMM: 91 P: 85 SS: 94 |
| 60 | 72 | SP: 100 HMM: 100 P: 100 SS: 99 | 293 | SP: 100 HMM: 100 P:100 SS: 100 | 103 | SP: 96 HMM: 96 P:98 SS: 98 | 101 | SP: 100 HMM: 98 P:98 SS: 97 |
| 100 | 72 | SP: 100 HMM: 100 P: 100 SS: 99 | 293 | SP: 100 HMM: 100 P: 100 SS: 100 | 103 | SP: 100 HMM: 100 P: 100 SS: 99 | 101 | SP: 100 HMM: 100 P: 100 SS: 99 |

**Table 2 Legend**

The number of sub-types for each family is shown in parenthesis along with the family names in the first row. The number of sequences in the alignment is shown in the first row under the family name for each family. To simulate the situation where a close homologue is not available, for each sequence (assumed to be of unknown sub-type) all other sequences with percentage identity greater than a threshold were ignored. The first column shows the similarity threshold used to eliminate sequences. For certain sequences (e.g. trypsins at 20%), the elimination process removes all sequences of the sub-type. In these situations, the sub-type of such a sequence is considered unpredictable. The first column, labeled "P" in each family shows the number of sequences for which a sub-type prediction was made. The second column for each family, labeled "A", gives the percentage accuracy of prediction for those sequences predicted. The four numbers given in this column are the accuracies for the *sub-profile based method* (SP), the *HMM based* (HMM), the *profile based method* (P) and the *similarity based method* (SS). For example, for the 191 kinases at threshold of 20%, the values are 100% (*sub-profile*), 94% (*HMM*) 96% (*profile*) and 60% (*similarity*). An "X" in the accuracy column means that no predictions could be made.

The *HMM* based *method* is almost always better than the *profile based.* This may indicate that the risk of wrong alignment by *hmmalign* is well compensated by the fact that *hmmalign* pays careful attention to the gap penalties which is not considered in the profile-based method where we only incorporate the "match" states in the profile. It may also reflect that only one of the four alignments studied here (the kinases) was hand-curated.

The *Sub-Profile based method* of this invention clearly outperforms all methods. Removing contributions from positions that do not discriminate between the sub-types has a dramatic effect.

Further Discussion of Figures

Figure 1: Alscript (Barton, 1993) figure showing an alignment of representatives of nucleotidyl cyclases with positions predicted to confer specificity to adenylate or guanylate highlighted by the method. The alignment only shows regions that contain positions predicted to confer specificity, deleted regions are indicated by dashes (---). Positions are highlighted only if the profile difference score for the residues was >0.5. Colours are according to the residue conservation: hydrophobics - yellow, small residues - light blue, positive residues - dark blue, negative residues - red, polar residues - magenta. Note that positions sharing the same colour across both groups may have subtle differences that are discussed in more detail in the text. Numbers above the alignment refer to positions discussed in the text, and refer to the PDB structure 1ab8.

Figure 2: Rasmol (Sayle & Milner-White, 1995) figure showing the structure of adenylate cyclase (PDB accession 1ab8, chain A), with positions found to confer specificity for adenylate or guanlyate by the method. Those that are starred (*) were reported to switch the specificity from guanylate to adenylate by mutagenesis (Tucker *et al.*, 1998).

Figure 3: Alscript (Barton, 1993) figure showing an alignment of representatives of protein kinases with positions predicted to confer specificity to Ser/Thr or Tyr highlighted by the method. Numbers above the alignment refer to positions discussed in the text, and refer to the PDB structure 2cpk. Labels below the alignment refer to the kinase "domain" nomenclature of Hanks et al (1988). Other details are as for Figure 1.

Figure 4: Rasmol (Sayle & Milner-White, 1995) figure showing the structure of cAMP dependent serine threonine kinase (PDB accession 2cpk, chain E), with positions found to confer specificity for serine/threonine or tyrosine by the method. Those that are starred (*) are taken from the literatures (Hanks & Hunter, 1988; Taylor et al 1995) and are thought to confer serine/threonine versus tyrosine specificity.

Figure 5: Alscript (Barton, 1993) figure showing an alignment of representatives of lactate and malate dehydrogenases with positions predicted to confer specificity lactate or malate highlighted by the method. Numbers above the alignment refer to positions discussed in the text, and refer to the PDB structure 9ldt. Other details are as for Figure 1.

Figure 6: Alscript (Barton, 1993) figure showing an alignment of representatives of tyrpsin like serine-proteases with positions predicted to confer specificites known for trypsins, chymotrypsins or elastases highlighted by the method. Numbers above the alignment refer to positions discussed in the text, and refer to the PDB structure 5ptp. The box shows a position not highlighted by the method that is thought to be involved in enzyme specificity. Other details are as for Figure 1.

References:

Andrade MA (1999) Position-specific annotation of protein function based on multiple homologs. *ISMB*, **7**, 28-33.

Andrade MA, Valencia A (1998) Automatic extraction of keywords from scientific text: application to the knowledge domain of protein families *Bioinformatics* **14**, 600-607.

Atrian S, Sanchez-Pulido L, Gonzalez-Duarte R, Valencia A (1998) Shaping of Drosophila alcohol dehydrogenase through evolution: relationship with enzyme functionality. *J. Mol. Evol.* **47**, 211-221.

Azuma Y, Renault L, Garcia-Ranea JA, Valencia A, Nishimoto T, Wittinghofer A (1999) Model of the ran-RCC1 interaction using biochemical and docking experiments. *J. Mol. Biol.* **289**, 1119-1130.

Bairoch A, Apweiler R (1999) The SWISSPROT protein sequence data bank and its new supplement TrEMBL in 1999. *Nucleic Acids Res* **27**, 49-54.

Barton GJ (1993) ALSCRIPT: a tool to format multiple sequence alignments. *Protein Eng.* **1**, 37-40.

Bateman A, Birney E, Durbin R, Eddy SR, Finn RD, Sonnhammer (1999) Pfam 3.1: 1313 multiple alignments and profile HMMs match the majority of proteins., *Nucleic Acids Res* **27**, 260-262.

Bauer B, Mirey G, Vetter IR, Garcia-Ranea JA, Valencia A, Wittinghover A, Carnonis JH, Cool RH (1999) Effector recognition by the small GTP-binding proteins Ras and Ral. *J. Biol. Chem*. **274**, 17763-17770.

Brenner SE (1999) Errors in Genome Annotation. *Trends Genet.* **15**, 132-133.

Casari G, Sander C, Valencia A (1995) A method to predict functional residues in proteins. *Nature Struct Biol* **2**, 171-178.

Chervitz SA, Aravind L, Sherlock G, Ball CA, Koonin EV, Dwight SS, Harris MA, Dolinski K, Mohr S. Smith T (1998) Comparison of the complete protein sets of woarm and yeast: orthology and divergence. *Science* **282**, 2022-2028.

Durbin R, Eddy S, Krogh A and Mitchison C (1998) *Biological sequence analysis: probabilistic models of protein and nucleic acids*, Cambridge University Press, Cambridge, UK.

Eddy SR (1998) Profile hidden Markov models, *Bioinformatics,* **14**, 755-763.

Gobel U, Sander C, Schneider R, Valencia A (1994) Correlated mutations and residue contacts in proteins, *Proteins: Struct Funct Genet,* **18**, 309-317.

Gribskov M, Luthey R, Eisenberg D (1990) *Methods Enzymol* **183**, 146-159

Hanks SK, Quinn AM, Hunter T (1988) The protein kinase family: conserved features and deduced phylogeny of the catalytic domains. *Science.* **241**, 42-52

Hanks SK, Hunter T (1996) The eukaryotic protein kinase superfamily: kinase (catalytic) domain structure and classification. *FASEB J.*, **9**, 576-596.

Hawrani AS, Sessions RB, Moreton KM, Holbrook JJ (1996) Guided Evolution of Enzymes with New Substrate Specificities. *J. Mol. Biol.* **264**, 97-110.

Krogh A, Brown M, Mian IS, Sjolander K, Haussler D (1994) Hidden Markov models in computational biology. Applications to protein modeling *J Mol Biol* **235**, 1501-1531

Lichtarge O, Bourne HR, Cohen FE (1996a) An evolutionary trace method defines binding surfaces common to protein families, *J Mol Biol,* **257**, 342-358.

Lichtarge O, Bourne HR, Cohen FE (1996b) Evolutionarily conserved G-alpha-beta-gamma binding surfaces support a model of the G protein-receptor complex, *Proc Natl Acad Sci USA* **93**, 7507-7511.

Lichtarge O, Yamamoto KR, Cohen FE (1997) Identification of functional surfaces of the zinc binding domains of intracellular receptors. *J Mol Biol* **274**, 325-337.

Livingstone CD, Barton GJ (1993) Protein sequence alignments: a strategy for the hierarchical analysis of residue conservation, *Comput Appl Biosci,* **9**, 745-756.

Makarova KS, Grishin NV (1999) The Zn-peptidase Superfamily: Functional convergence after evolutionary divergence. *J. Mol. Biol.* **292**, 11-17.

Murzin, A.G. (1993) Can homologous proteins evolve different enzymatic activities? *Trends Biochem Sci,* **18**, 403-405.

Neher E (1994) How frequent are correlated changes in families of protein sequences? *Proc Natl Acad Sci USA,* **91**, 98-102.

Olmea O, Valencia A (1997) Improving contact predictions by the combination of correlated mutations and other sources of sequence information. *Fold Des* **2**, S25-35

Pazos F, Hlmer-Citterich M, Ausiello G, Valencia A (1997) Correlated mutations contain information about protein-protein interaction. *J Mol Biol*, **271**, 511-523.

Pellegrini M, Marcotte EM, Thompson MJ, Eisenberg D, Yeates TO (1999) Assigning protein functions by comparative genome analysis: protein phylogenetic profiles *Proc Natl Acad Sci U S A* **96**, 4285-4288

Rossmann MG, Argos P (1976) Exploring structural homology in proteins. *J. Mol. Biol*. **25**, 75-95.

Russell RB, Barton GJ (1992) Multiple protein sequence alignment from tertiary structure comparison: assignment of global and residue confidence levels. *PROTEINS: Struct Funct Genet* **14,** 309-323.

Russell RB, Barton GJ (1994) Structural features can be unconserved in proteins with similar folds: An analysis of side-chain to side-chain contacts, secondary structure and acessibility. *J. Mol. Biol.* **244**, 332-350.

Russell RB, Sasieni PD, Sternberg MJE (1998) Supersites within superfolds: binding site similarity in the absence of homology *J Mol Biol* **282**, 903-918.

Sayle RA, Milner-White EJ (1995) RASMOL: Biomolecular graphics for all. *Trends Biochem Sci* **20**, 374.

Shannon C, Weaver W (1963) *Mathematical Theory of Communication*, University of Illinois press, Champaign, IL, USA.

Schultz J, Milpetz F, Bork P, Ponting CP (1998) SMART, a simple modular architecture research tool: identification of signalling domains, *Proc Natl Acad Sci* **95**, 5857-5864.

Smith CM, Shindyalov IN, Veretnik S, Gribskov M, Taylor SS, Ten Eyck LF, Bourne PE (1997) The protein kinase resource *Trends Biochem Sci* **22**, 444-446.

Swindells MB, MacArthur MW, Thornton JM (1995) Intrinsic phi, psi propensities of amino acids, derived from the coil regions of known structures. *Nat Struct Biol* 2, 596-603.

Taylor WR (1986) The classification of amino acid conservation. *J. Theor Biol,* **119**, 205-218.

Taylor SS, Radzio-Andzelm E, Hunter T (1995) How do protein kinases discrminate between serine/threonine and tyrosine? Structural insights from the insulin receptor protein tyrosine kinases. *FASEB J.* **9**, 1255-1266.

Taylor WR, Hatrick K (1994) Compensating changes in protein multiple sequence alignments. *Protein Eng*, **7**, 341-348.

Tucker CL, Hurley JH, Miller TR, Hurley, JB (1998) Two amino acid substitutions convert a guanylyl cyclase, RetGC-1, into an adenylyl cyclase. *Proc. Nat. Acad. Sci* (USA), **11**, 5994-5997.

Wilks HM, Hart KW, Feeney R, Dunn CR, Muirhead H, Chia WN, Barstow DA, Atkinson T, Clarke AR, Holbrook JJ (1988) A specific, highly acitve malate dehydrogenase by redesign of a lactate dehydrogenase framework. *Science* **242**, 1541-1544.

Zhang G, Liu Y, Ruoho AE, Hurley JH (1998) Structure of the adenylyl cyclase catalytic core. *Nature*, **386**, 247-253.

Zvelebil MJJ, Barton GJ, Taylor WR, Sternberg MJE (1987) Prediction of protein secondary structure and active sites using the alignment of homologous sequences. *J. Mol. Biol.* **195**, 957-961.

**Claims**

**1.**    A process for identifying one or more positions which discriminate between different sub-types according to some definition of function in a family of proteins, the process comprising, in a computational device:

> Constructing a set of multiple aligned protein sequences from said family wherein one set of sequences has one known sub-type and one or more other set of the sequences each has a different known sub-type;
> constructing profile for each sub-type
> calculating positional entropy of each position for each sub-type relative to the other sub-types
> converting the positional relative entropies to positional z-scores
> identifying positions which discriminate between the sub-types as the positions with high z-scores

**2.**    A process for identifying one or more positions which discriminate between different sub-types according to some definition of function in a large family of proteins the process comprising identifying one or more positions which have a *z-score* greater than about 3.0 wherein the *z-score* is calculated by the process comprising:

> in an alignment $A$ of sequences in family $F$, which have sub-types $S_1$, $S_2$, • • •, $S_k$ of the sequences,
> extracting the sub-alignment $A^j$ from $A$, corresponding to the sequences of sub-type $S_j$;
> building profile $P^j$ of the alignment $A^j$ wherein the profile at position $i$ of the alignment is represented by $P^j_i$ and the profile value for amino acid $x$ at position $i$ of the alignment is represented by $P^j_{i,x}$ using the *hmmbuild* program of the HMMER 2.1.1 suite;
> assigning the identifier $\bar{s}$ to a sub-type s to denote the union of all the sub-types excluding s;
> computing the relative entropy of the position $i$ for sub-type s with respect to the entropy of that position for the sub-type $\bar{s}$ by making $RE^s_i$ be the relative entropy of $P^s_i$ with respect to $P^{\bar{s}}_i$ according to the formula;

$$RE^s{}_i = \sum_{\text{for all } x} P^s{}_{i,x} \log \frac{P^s{}_{i,x}}{P^{\bar{s}}{}_{i,x}}$$

> calculating a cumulative relative entropy for position $i$ in all sub-types according to the formula

$$CRE_i = \sum_{\text{for all sub-types } s} RE^s{}_i$$

> converting the cumulative relative entropy for position $i$ into a *z-score* using the formula

$$Z_i = \frac{CRE_i\text{-}\mu}{\sigma}$$

wherein $\mu$ and $\sigma$ are the mean and the standard deviation of cumulative relative entropies of all positions.

3. A method for predicting the subtype or subtypes of a new protein, which is related to a family of proteins wherein, the method comprises:

identifying one or more positions which discriminate between different sub-types according to some definition of function in said family by determining one or more positions in pre-aligned sequences of said family and the new protein which have a *z-score* greater than about 3.0 wherein the *z-score* is calculated by:
in an alignment $A$ of sequences in family $F$, which have sub-types $S_1$, $S_2$, $\cdot\cdot\cdot$, $S_k$ of the sequences, extracting the sub-alignment $A^j$ from $A$, corresponding to the sequences of sub-type $S_j$;
building profile $P^j$ of the alignment $A^j$ wherein the profile at position $i$ of the alignment is represented by $P^j_i$ and the profile value for amino acid $x$ at position $i$ of the alignment is represented by $P^j_{i,x}$ using the *hmmbuild* program of the HMMER 2.1.1 suite;
assigning the identifier $\bar{s}$ to a sub-type s to denote the union of all the sub-types excluding s;
computing the relative entropy of the position $i$ for sub-type s with respect to the entropy of that position for the sub-type $\bar{s}$ by making $RE^s_i$ be the relative entropy of $P^s_i$ with respect to $P^{\bar{s}}_i$ according to the formula;

$$RE^s_i = \sum_{\text{for all } x} P^s_{i,x} \log \frac{P^s_{i,x}}{P^{\bar{s}}_{i,x}}$$

calculating a cumulative relative entropy for position $i$ in all sub-types according to the formula

$$CRE_i = \sum_{\text{for all sub–types } s} RE^s_i$$

converting the cumulative relative entropy for position $i$ into a *z-score* using the formula

$$Z_i = \frac{CRE_i\text{-}\mu}{\sigma}$$

wherein $\mu$ and $\sigma$ are the mean and the standard deviation of cumulative relative entropies of all positions;
computing the profile score of the pre-aligned sequence of the unknown sub-type for each possible sub-type by considering only the positions which have a z-score of greater than 0; and
assigning the unknown protein to the sub-type yielding the highest z-score.

FIG. 1A

CYA1_BOVIN
CYA1_DROME
CYA2_HUMAN
CYA2_RAT
CYA3_RAT
CYA4_RAT
CYA5_RABIT
CYA6_CANFA
CYA6_RAT
CYA7_MOUSE
CYA8_RAT
CYAA_ANACY
CYA1_BOVIN
CYA5_CANFA
CYA5_RAT
CYA6_MOUSE
CYA1_DROME
CYA2_HUMAN
CYA2_RAT
CYA4_RAT
CYA7_HUMAN
CYA3_RAT
CYA8_RAT
CYAA_DICDI
1ab8a

```
                 1014      1018    1019-20
CYA1_BOVIN    G  L  R [K] W  Q  Y [D]     [V] W  S  N  D  V  T
CYA1_DROME    G  A  R  K  P  Q  Y  D       I  W  G  N  T  V  N
CYA2_HUMAN    G  A  Q  K  P  Q  Y  D       I  W  G  N  T  V  N
CYA2_RAT      G  A  Q  K  P  Q  Y  D       I  W  G  N  T  V  N
CYA3_RAT      G  A  R  K  P  H  Y  D       I  W  G  N  T  V  N
CYA4_RAT      G  A  Q  K  P  Q  Y  D       I  W  G  N  T  V  N
CYA5_RABIT    G  L  R  K  W  Q  F  D       V  W  S  N  D  V  T
CYA6_CANFA    G  L  R  K  W  Q  F  D       V  W  S  N  D  V  T
CYA6_RAT      G  L  R  K  W  Q  F  D       V  W  S  N  D  V  T
CYA7_MOUSE    G  L  R  K  W  Q  Y  D       V  W  S  H  D  V  S
CYA8_RAT      G  L  R [K] W  Q  F  D       V [W] S  W  D  V  D
CYAA_ANACY    G  F  T [S]    R  I  D  S  T  V [I] G  D  A  V  N  ---
CYA1_BOVIN    G  A  R [R]    Q  Y  D       I [W] G  N  T  V  N
CYA5_CANFA    G  A  R  K  P  Q  Y  D       I  W  G  N  T  V  N
CYA5_RAT      G  A  R  K  P  Q  Y  D       I  W  G  N  T  V  N
CYA6_MOUSE    G  A  R  K  P  Q  Y  D       I  W  G  N  T  V  N
CYA1_DROME    G  A  R  K  P  Q  Y  D       I  W  G  N  T  V  N
CYA2_HUMAN    G  A  Q  K  P  Q  Y  D       I  W  G  N  T  V  N
CYA2_RAT      G  A  Q  K  P  Q  Y  D       I  W  G  N  T  V  N
CYA4_RAT      G  A  Q  K  P  Q  Y  D       I  W  G  N  T  V  N
CYA7_HUMAN    G  A  R  K  P  Q  Y  D       I  W  G  N  T  V  N
CYA3_RAT      G  A  R  K  P  H  Y  D       I  W  G  N  T  V  N
CYA8_RAT      G  A  K  K  P  Q  Y  D       I  W  G  K  T  V  N
CYAA_DICDI    G  I  S  R  P  K  F  D       V  W  G  D  T  A  N
1ab8a         G  A  Q [K] P  Q  Y [D]     [I][W] G  N  T  V  N
```

## FIG. 1B

```
                                     890                        919                         937-8
ANPA_HUMAN         F D S V T I Y F [S] D I     T L L N D L [Y] T C F D A     V Y K [V] [E] T I G D A Y
ANPA_RAT           F D S V T I Y F [S] D I     T L L N D L [Y] T C F D A     V Y K [V] [E] T I G D A Y
ANPB_BOVIN         F D S V T I Y F [S] D I     T L L N D L [Y] T C F D A     V Y K [V] [E] T I G D A Y
ANPB_RAT           F D S V T I Y F [S] D I     T L L N D L [Y] T C F D A     V Y K [V] [E] T I G D A Y
CYG3_BOVIN         F G N V T M L F [S] D I     T M L N A L [Y] T R F D R     V Y K [V] [E] T I G D A Y
CYG3_RAT           F N E V T M L F [S] D I     T M L N A L [Y] T R F D Q     V Y K [V] [E] T I G D A Y
CYG5_HUMAN   - - - F S N V T M L F [S] D I --- T M L N A L [Y] T R F D Q --- V Y K [V] [E] T I A M P I  ---
CYGD_HUMAN         F E Q V T L Y F [S] D I     D L L N D L [Y] T L F D A     V Y K [V] [E] T I G D A Y
CYGE_RAT           F E E V T L Y F [S] D I     D L L N D L [Y] T L F D A     V Y K [V] [E] T I G D A Y
CYGF_RAT           F D L V T L Y F [S] D I     D L L N D L [Y] T L F D A     V Y K [V] [E] T I G D A Y
CYGS_STRPU         F E M V S I F F [S] D I     N L L N D L [Y] T L F D A     V Y K [V] [E] T I G D A Y
HSER_HUMAN         Y E E V T I Y F [S] D I     D M L N D I [Y] K S F D H     V Y K [V] [E] T I G D A Y
HSER_RAT           Y E E V T I Y F [S] D I     D M L N D I [Y] K S F D Q     V Y K [V] [E] T I G D A Y
CYG1_BOVIN         Y D N V T I L F [S] G I     N L L N D L [Y] T R F D T   F V Y K [V] [E] T V G D K Y
CYG1_HUMAN         Y D N V T I L F [S] G I     N L L N D L [Y] T R F D T   F V Y K [V] [E] T V G D K Y
CYG1_RAT           Y D N V T I L F [S] G I     N L L N D L [Y] T R F D T   F V Y K [V] [E] T V G D K Y
```

## FIG. 1C

```
                    1014      1018   1019-20
ANPA_HUMAN    G  L  K  M  P  R  Y  C    L  F  G  D  T  V  N
ANPA_RAT      G  L  K  M  P  R  Y  C    L  F  G  D  T  V  N
ANPB_BOVIN    G  L  K  M  P  R  Y  C    L  F  G  D  T  V  N
ANPB_RAT      G  L  K  M  P  R  Y  C    L  F  G  D  T  V  N
CYG3_BOVIN    G  V  K  M  P  R  Y  C    L  F  G  N  N  V  T
CYG3_RAT      G  V  K  M  P  R  Y  C    L  F  G  N  N  V  T
CYG5_HUMAN    G  V  K  M  P  R  Y  C    L  F  G  N  N  V  T  - - -
CGYD_HUMAN    G  L  T  M  P  R  Y  C    L  F  G  D  T  V  N
CGYE_RAT      G  L  T  M  P  R  Y  C    L  F  G  D  T  V  N
CYGF_RAT      G  L  T  M  P  R  Y  C    L  F  G  D  T  V  N
CYGS_STRPU    G  L  T  M  P  R  Y  C    L  F  G  D  T  V  N
HSER_HUMAN    G  I  K  M  P  R  Y  C    L  F  G  D  T  V  N
HSER_RAT      G  I  K  M  P  R  Y  C    L  F  G  D  T  V  N
CYG1_BOVIN    G  Q  R  M  P  R  Y  C    L  F  G  D  T  V  N
CYG1_HUMAN    G  Q  R  M  P  R  Y  C    L  F  G  D  T  V  N
CYG1_RAT      G  Q  R  M  P  R  Y  C    L  F  G  D  T  V  N
```

# FIG. 1D

FIG. 2

```
                158              168-70           201 203-4      209        229          273
TPK1      Y L H S K D I I Y R D L K P E N I L   L C G T P D Y I A P E V V   G I L I Y E   L L S R L I T R D
PsPK5     Y L H C Q G I I Y R D L K P E N V L   F V G T E E Y I A P E I I   G I L L Y E   L I Y Y L L Q R D
DC0       Y L H Y L D L I Y R D L K P E N L L   L C G T P E Y L A P E I I   G V L V Y E   L L R N L L Q V D
CamKI     Y L H D L G I V H R D L K P E N L L   A C G T P G Y V A P E V L   G V I A Y I   F I R H L M E K D
MAK       F I H K H G F F H R D M K P E N L L   Y V S T R W Y R A P E V L   G S I M A E   V V K K C L R W D
DdPK1     Y L H A E N I I Y R D L K P E N I L   S C G T F D Y M A P E I L   G V V V Y E   F I F Q L L S V D
PhKgT     F L H A N N I V H R D L K P E N I L   L C G T P G Y L A P E I L   G V I L F T   L I S R L L Q V D
PKCe      F L H Q H G V I Y R D L K L D N I L   F C G T P D Y I A P E I L   G V L M Y E   I L K A F M T K N
SNF1      Y C H R H K I V H R D L K P E N L L   S C G S P N Y A A P E V I   G V I L Y V   L I K R M L I V N
RKIN1     Y C H R N K V V H R D L K P E N L L   S C G S L N Y A A P E V I   G V I L Y A   L I S R M L I V D
prp1+     L L K Q C N V I H S D I K P D N M L   Y L V S R F Y R A P E I I   G C S L Y E   L M N K F L T Y F
DdMLCK    Y L H G L N I V H R D L K P E N L L   A C G T P S Y V A P E V L   G V I T Y I   F I G K L L V V D
FpCKII    Y C H S Q G I M D R D V K P H N V M   R V A S R Y F K G P E L L   G C M F A G   F L D K L L R Y D
AKIN10    Y C H R N M V V H R D L K P E N I L   S C G S P N Y A A P E V I   G V I L Y A   L I P R M L V V D
B.emC     Y L H S K D I I Y R D L K P E N L L   L C G T P D Y L A P E I I   G V L I F E   L L K R L L T T D
DmERKa    Y I H S A N V L H R D L K P S N L L   Y V A T R W Y R A P E I M   G C I L E A   L L G K M L T F N
Sgk       Y L H S L N I V Y R D L K P E N I L   F C G T P E Y L A P E V L   G A V L Y E   L L E G L L Q K D
Cdk6      F L H S H R V V H R D L K P Q N I L   V V V T L W Y R A P E V L   G C I F A E   L L L K C L T F N
```

## FIG. 3A

FIG. 3B

```
                  158              168-70            201 203-4     209          229          273
RET      Y L A E M K L V H R D L A A R N I L   G R I P V K W M A I E S L   G V L L W E   L M Q R C W A P S
Btk      Y L E S K Q F L H R D L A A R N C L   S K F P V R W S P P E V L   G V L M W E   V M K L C W S H G
Ror2     Y L S S H H V V H K D L A T R N V L   S L L P I R W M P P E A I   G V V L W E   L M I E C W H E Q
LCK      F I E E R N Y I H R D L R A A N I L   A K F P I K W T A P E A I   G I L L T E   I I T E C W R G R
Dtrk     A I Y R A R F T H R D L A T R N C V   T L L P I R W L A P E C I   G V V V W E   L I Q K M L Q T D
JAK2     Y L G T K R Y I H R D L A T R N I L   G E S P I F W Y A P E S L   G V V L Y E   L M Q L C W H A T
ECK      Y L A N M N Y V H R D L A A R N I L   G K I P I R W T A P E A I   G I V M W E   L M L D C W H K D
c-ros    Y L E R M H F I H R D L A A R N C L   G L L P V R W M A P E S L   G I L I W E   L L L L C W R T D
HEK2   ---Y L S E M N Y V H R D L A A R N I L---G K I P I R W T A P E A I---G I V M W E---L M L D C W Q K D---
HOP      Y L S D M G L I H R D L A A R N I L   R D I P I R W Y S P E A I   G V T L F E   V M V Q C W A H K
c-Yes    Y I E R M N Y I H R D L R A A N I L   A K F P I K W T A P E A A   G I L Q T E   L M I H C W K K D
c-Src    Y V E R M N Y V H R D L R A A N I L   A K F P I K W T A P E A A   G I L L T E   L M N L C W K K D
Nkin15   Y L A S I P C V H R D L A L R N V L   T P M P A R W M A P E V M   G V S L Y E   L M K L C W H E K
Nuk      Y L A D M N Y V H R D L A A R N I L   G K I P I R W T A P E A I   G I V M W E   L M K N C W A Y D
c-Sea    Y L A Q K K F V H R D L A A R N C M   A K L P V K W M A L E S L   G V L M W E   V M Q Q C W E A D
HEK      Y L S D M G Y V H R D L A A R N I L   G K I P I R W T S P E A I   G I V L W E   L M L D C W Q K D
ErbB4    Y L E E R R L V H R D L A A R N V L   G K M P I K W M A L E C I   G V T I W E   T L L S C W H L D
INS.R    Y L N A K K F V H R D L A A R N C M   G L L P V R W M A P E S L   G V V L W E   L M S R C W Q P N
Flk2     F L E F K S C V H R D L A A R N V L   A R L P V K W M A P E S L   G I L L W E   I M L D C W H R D

sub-domain               VI                        VIII               IX           XI
```

FIG. 3C

FIG. 4

EP 1 096 411 A2

```
                      102        107      162 164          190   (194)
LDH_DEIRA    A  N [Q]  K  P  G [E] S   I [G] S [G] T  V  L  D   [Y] V  L  G [E] H  G  D
LDH_TOXGO    L  T  K   V  P  G  K  S   C [G] M  A  N  V  L  D    T  V  I  G  T  H  G  D
LDH_MYCPN    R  P [Q] K Q  G  G [E] T  I [G] S [G] T  L  L  D   [Y] V  L  G [E] H  G  D
LDH_DROME    V  R [Q]  K  E  G [E] S   I [G] S [G] T  N  L  D   [W] I  I  G [E] H  G  D
LDH_STRTR    A  P [Q]  K  P  G [E] T   I [G] S [G] T  S  L  D    Y  I  M  G [E] H  G  D
LDH_MYCHY    R  P [Q]  K  P  G [E] T   I [G] S [G] T  V  L  D    Y  V  M  G [E] H  G  D
LDHB_HORVU   A  R [Q]  I  P  G [E] T   I [G] S [G] T  N  L  D   [Y] M  V  G [E] H  G  D
LDHC_XENLA   V  R [Q]  Q  E  G [E] S   I [G] S [G] T  N  L  D    F  I  L  G [E] H  G  D
LDHX_MOUSE   A  R  M   V  S  G [E] T   I [G] S [G] C  N  L  D   [W] V  L  G [E] H  G  D
LDH_BACSU    A  N [Q]  K  P  G [E] T   I [G] S [G] T  T  L  D    H  I  I  G [E] H  G  D
LDH_MYCGE    R  P  Q K Q  G  G [E] T   I [G] S [G] T  L  L  D   [Y] V  I  G [E] H  G  D
LDH_THEAQ    V  A  Q   R  P  G [E] T   V [G] S [G] T  I  L  D    Y  V  V  G [E] H  G  D
LDHH_CHICK   V  R  Q   Q  E  G [E] S   I [G] S [G] C  N  L  D    W  I  L  G [E] H  G  D
LDH_LACLA    ---A  P  Q   K  P  G [E] T---V [G] S [G] T  S  L  D---Y  I  M  G [E] H  G  D---
LDH_STRMU    A  P  Q   K  P  G [E] T   I [G] S [G] T  S  L  D    Y  I  M  G [E] H  G  D
LDHM_MOUSE   A  R  Q   Q  E  G [E] S   I [G] S [G] C  N  L  D    W  V  L  G [E] H  G  D
LDHB_FUNHE   V  R  Q   Q  E  G [E] S   I [G] S [G] T  N  L  D    W  V  L  G [E] H  G  D
LDH_PETMA    A  R [Q]  Q  E  G [E] S   I [G] S [G] C  N  L  D   [W] I  I  G [E] H  G  D
LDH_METJA    V  P  R   K  E  G  M  S   F [G] L  G  T  H  L  D    R  I  I  G [E] H  G  D
LDH_BACCA    A  N [Q]  K  P  G [E] T   I [G] S [G] T  I  L  D   [Y] I  I  G [E] H  G  D
LDHB_FUNPA   V  R [Q]  Q  E  G [E] S   I [G] S [G] T  N  L  D    W  V  L  G [E] H  G  D
LDH_LACPL    A  P [Q]  K  P  G [E] S   I [G] S [G] T  S  L  D    Y  I  M  G [E] H  G  D
LDHM_BOVIN   A  R [Q]  Q  E  G [E] S   I [G] S [G] C  N  L  D   [W] I  L  G [E] H  G  D
LDH1_PLAFD   F  T  K   A  P  G  K  S   I [G] L  G  G  V  L  D    H  I  V  G  A  H  G  N
LDH_STRPN    A  P [Q]  K  P  G [E] T   I [G] S [G] T  S  L  D   [Y] I  M  G [E] H  G  D
LDHH_RAT     V  R [Q]  Q  E  G [E] S   I [G] S [G] C  N  L  D    W  I  L  G [E] H  G  D
LDHX_HUMAN   A  R [Q]  Q  E  G [E] T   I [G] S [G] C  N  L  D    W  I  I  G [E] H  G  D
LDHH_MOUSE   V  R [Q]  Q  E  G [E] S   I [G] S [G] C  N  L  D    W  I  L  G [E] H  G  D
9ldta        A  R [Q]  Q  E  G [E] S   I [G] S [G] C  N  L  D   [W] I  L  G [E] H  G  D
```

## FIG. 5A

```
                       102        107     162 164          190   (194)
MDHM_PIG        V P  R   K P G  M  T    I  F  G  V  T T L D    P  V I G  G  H A G
MDHG_BRANA      V P  R   K P G  M  T    L  L  G  V  T T L D    P  V V G  G  H A G
MDH_BACIS       I A  R   K P G  M  S    I  G  Q  S  G V L D    F  V L G  G  H G D
MDHG_CITVU      V P  R   K P G  M  T    L  L  G  V  T M L D    P  V V G  G  H A G
MDHM_CITVU      V P  R   K P G  M  T    L  F  G  V  T T L D    P  V I G  G  H A G
MDH_BACSU       I A  R   K P G  M  S    I  G  Q  S  G V L D    F  V L G  G  H G D
MDH_CHLAU       A P  R   K P G  M  S    I  G  Q  A  G V L D    M  L M G  G  H G D
MDHP_YEAST   ---V P  R   K P G  L  T---V  M  G  V  T N L D---T V  I G  G  H S G---
MDHM_EUCGU      V P  R   K P G  M  T    L  L  G  V  T T L D    P  V V G  G  H A G
MDH_HALMA       I P  R   Q P G  Q  T    I  G  F  G  G R L D    T  I L G E H G D
MDH_HAEIN       V A  R   K P G  M  D    L  F  G  V  T T L D    P  V I G  G  H S G
MDHC_PIG        M P  R   R D G  M  E    F  S  C  L  T R L D    I  I W G N H S S
MDHM_MOUSE      V P  R   K P G  M  T    I  F  G  V  T T L D    P  V I G  G  H A G
MDHG_SOYBN      V P  R   K P G  M  T    L  L  G  V  T M L D    P  V V G  G  H A G
MDH_CHLTE       L P  R   K P G  M  T    I  G  M  A  G V L D    C  V L G  G  H G D
MDH_THEFL       A P  R   K A G  M  E    F  T  A  M  T R L D    T  V W G N H S S
MDHM_CAEEL      V P  R   K P G  M  T    V  F  G  V  T T L D    P  V V G  G  H A G
```

## FIG. 5B

FIG. 6A

```
                      (216)        221           226
EL3A_HUMAN    S F |V| S G F G C |N| F I W K P |T| V F T R V
EL3B_HUMAN    S F |V| S A F G C |N| T R R K P |T| V F T R V
EL2A_HUMAN    S F |G| S R L G C |N| Y Y H K P  S  V F T R V
EL2B_HUMAN    S L |T| S V L G C |N| Y Y Y K P  S  I F T R V
EL2_BOVIN     S F |G| S S L G C |N| Y Y R K P  S  V F T R V---
EL2_PIG       S F |G| S S L G C |N| Y Y H K P  S  V F T R V
EL2_MOUSE     S F |G| S S L G C |N| Y P R K P  S  V F T R V
EL2_RAT       S F |G| S T L G C |N| Y P R K P  S  V F T R V
EL1_BOVIN     S F |V| S S L G C |N| V S K K P |T| V F T R V
EL1_RAT       S F |V| S S M G C |N| V S K K P |T| V F T R V
EL1_PIG       S F |V| S R L G C |N| V T R K P |T| V F T R V

                      (216)        221           226
CTR1_ANOGA    N F |G|     V P C A L G    Y P D G F A R V
CTR2_ANOGA    N F |G|     V P C G R G    F P D G F A R V
CTR2_VESCR    S Y |G|     H P C A V G    S P N V F T R V
CTR2_VESOR    S Y |G|     H P C A I G    S P N V F T R V
CTR2_CANFA    S W |G|     S G T C S T S  T P G V Y A R V
CTRB_BOVIN    S W |G| S   S T C S T S    T P A V Y A R V
CTRB_HUMAN    S W |G| S   D T C S T S    S P G V Y A R V
CTRB_RAT      S W |G|     S G V C S T S  T P A V Y S R V---
CTRA_BOVIN    S W |G| S   S T C S T S    T P G V Y A R V
CTRA_GADMO    S W |G|     S G T C T P T  M P G V Y A R V
CTRB_GADMO    S W |G| S   S R C S V T    T P A V Y A R V
CTRL_HUMAN    S W |G| T   K N C N V R    A P A V Y T R V
CTRL_HALRU    S W |G| I   S S C S G S    Y P S V Y T R V
CTR1_PENVA    S F |G| S S A G C E K G    Y P A A F T R V
CTR2_PENVA    S F |G| S S A G C E V G    Y P D A F T R V
```

## FIG. 6B

30

EP 1 096 411 A2

```
              121                    137                          184            189
TRY1_BOVIN    R V A S[I]S L P T S C A    S A G T Q C L I S G W G N    I T S N M F C A G Y L    E G G K  [D]S C Q G D S
TRYA_MANSE    A A Q P A R I A G A N Y N L G D N Q V V[W]A A G W G A    V T D N M L C S G W L D V G G R  [D]Q C Q G D S
TRYC_MANSE    A A Q P A R I A G A N Y N L G D N Q V V[W]A A G W G A    V T D N M L C S G W L D V G G R  [D]Q C Q G D S
TRYM_RAT      H I H P[I]S L P P A S E T F P S G T S C[W]V T G W G D    V Q D G M L C A G N        T R S  [D]S C Q G D S
TRYB_HUMAN    H V H T V T L P P A S E T F P P G M P C[W]V T G W G D    V R D D M L C A G N        T R R  [D]S C Q G D S
TRYT_CANFA    H V Q P V T L P P A L Q T F P T G T P C[W]V T G W G D    V R E D M L C A G N        S K S  [D]S C Q G D S
TRYP_ASTFL    N V A P[I]A L P A Q G H    T A T G  N V I V T G W G T    I F D S M I C A G V P    E G G K  [D]S C Q G D S
TRYU_DROER    T M E A[I]E I A A E Q P    A V G V Q A T I S G W G Y    I S E G M L C A G L S    E G G K  [D]A C Q G D S
TRYZ_DROER    T I K A[I]K L A T E P P    L D G A P S K I S G W G S    I T S A M L C A G K R G V G G A  [D]A C Q G D S
TRYD_DROME    T I K A[I]G L A S S N P    A N G A A G S V S G W G T    I R S T M I C A A A       S G K  [D]A C Q G D S
TRYB_DROME    T I K A[I]G L A S S N T    A N G A A A S V S G W G T    I K S S M I C A F A       S G K  [D]S C Q G D S
TRY3_LUCCU    T I K T[I]G L A T A A P    A N G A A A T V S G W G T    I K A S M I C A Y T       V G K  [D]S C Q G D S
TRYE_DROME    S I R E[I]R I A D S N P    R E G A T A V V S G W G T    I K D T M L C A Y A       P H K  [D]A C Q G D S
TRY1_ANOGA    S V Q P V G L P K Q D E T V K D G T M T T V S G W G N    V T D R M L C A G Y Q    Q G G K  [D]A C Q G D S
TRY6_ANOGA  ---N L Q P V S L P E Q D D P I E E G T M G I V S G W G M---I T E Q M F C A G Y K    Q G G T   G T C R N D S---
TRYP_SIMVI    K T A A[I]E L A E V G E E V E T D A M A I V S G W G D    V T E R M I C A G Y L    A G G R  [D]S C Q G D S
TRYP_STRGR    P T L K[I]A T T T A Y N    Q G   T F T V A G W G A    V A N E E I C A G Y P    D T G G V[D]T C Q G D S
TRY4_HUMAN    R V S T[I]S L P T A P P    A A G T E C L I S G W G N    I T N S M F C V G F L    E G G K  [D]S C Q R D S
TRY1_HUMAN    R V S T[I]S L P T A P P    A T G T K C L I S G W G N    I T S N M F C V G F L    E G G K  [D]S C Q G D S
TRY1_RAT      R V A P V A L P S A C A    P A G T Q C L I S G W G N    I T S S M I C V G F L    E G G K  [D]S C Q G D S
TRYP_MOUSE    R V A S V P L P S S C A    P A G T Q C L I S G W G N    I T N N M I C V G F L    E G G K  [D]S C Q G D S
TRY2_CANFA    R V A T[I]S L P R A C A    A P G T Q C L I S G W G N    I T E N M I C A G F L    E G G K  [D]S C Q G D S
TRYP_PIG      R V A T V S L P R S C A    A A G T E C L I S G W G N    I T G N M I C V G F L    E G G K  [D]S C Q G D S
TRY3_CHICK    D I Q P[I]A L P S S C A    K A G T E C L I S G W G N    I T S N M I C V G F L    E G G K  [D]S C Q G D S
TRY2_XENLA    N I Q S V P L P S A C A    S A G T N C L I S G W G N    I T K N M F C A G F L    A G G K  [D]S C Q G D S
TRY1_GADMO    Y V H A V A L P T E C A    A D A T M C T V S G W G N    I T Q S M F C A G Y L    E G G K  [D]S C Q G D S
TRY1_SALSA    Y V Q P V A L P T S C A    P A G T M C T V S G W G N    I T N A M F C A G Y L    E G G K  [D]S C Q G D S
TRYP_SQUAC    N V D L[I]S L P T G C A    Y A G E M C L I S G W G N    I T N N M M C V G Y M    E G G K  [D]S C Q G D S
TRYA_RAT      K V S T[I]P L P Q Y C P    T A G T E C L V S G W G V    I T N N M F C L G F L    E G G K  [D]S C Q Y D S
TRYP_PLEPL    A V A P[I]P L P T S C P    V A G T P C S V S G W G N    I S P R M V C A G F M    D G S R  [D]A C N G D S
5ptp          R V A S[I]S L P T S C A    S A G T Q C L I S G W G N    I T S N M F C A G Y L    E G G K  [D]S C Q G D S
```

# FIG. 6C

```
TRY1_BOVIN    S W G    S G C A Q K N K P G V Y T K V
TRYA_MANSE    S W G    E E C A L A R F P G V N A R V
TRYC_MANSE    S W G    E E C A L A R F P G V N A R V
TRYM_RAT      S W G    E G C A E A N R P G I Y T R V
TRYB_HUMAN    S W G    E G C A Q P N R P G I Y T R V
TRYT_CANFA    S W G    E G C A Q P N R P G I Y T R V
TRYP_ASTFL    S W G    Y G C A R P G Y P G V Y T E V
TRYU_DROER    S W G    E G C A R P N Y P G V Y A N V
TRYZ_DROER    S W G    N S C A L P N Y P G V Y A N V
TRYD_DROME    S W G    Y G C A Y S N Y P G V Y S D V
TRYB_DROME    S W G    Y G C A A A N Y P G V Y A D V
TRY3_LUCCU    S W G    Y G C A A V N Y P G V Y A D V
TRYE_DROME    S W G    Y G C G D V R Y P G V Y A D V
TRY1_ANOGA    S W G    Y G C A Q A G Y P G V Y S R V
TRY6_ANOGA    S W G    H E C E L A G Y P G V Y A R V---
TRYP_SIMVI    S W S    V G C A Q S N F P G V Y G
TRYP_STRGR    S W G    Y G C A R P G Y P G V Y T E V
TRY4_HUMAN    S W G    H G C A W K N R P G V Y T K V
TRY1_HUMAN    S W G    D G C A Q K N K P G V Y T K V
TRY1_RAT      S W G    Y G C A L P D N P G V Y T K V
TRYP_MOUSE    S W G    Y G C A Q P D A P G V Y T K V
TRY2_CANFA    S W G    Y G C A Q K N K P G V Y T K V
TRYP_PIG      S W G    Y G C A Q K N K P G V Y T K V
TRY3_CHICK    S W G    I G C A L K G Y P G V Y T K V
TRY2_XENLA    S W G    Y G C A Q R N Y P G V Y T K V
TRY1_GADMO    S W G    Y G C A E R D H P G V Y A K V
TRY1_SALSA    S W G    Y G C A E P G N P G V Y A K V
TRYP_SQUAC    S W G    Y G C A E R D H P G V Y T R V
TRYA_RAT      S W G    D G C A L E G K P G V Y T K V
TRYP_PLEPL    S W G    Q G C A Q P N Y P G V Y V K L
5ptp          S W G    S G C A Q K N K P G V Y T K V
```

# FIG. 6D